# EUROPEAN PATENT APPLICATION

(11) **EP 3 804 756 A1**
(43) Date of publication of application: **14.04.2021**
(21) Application number: 19808289.3
(22) Date of filing: 24.05.2019
(51) Int. Cl.: A61K 45/00, A23L 33/10, A61K 31/194, A61K 33/10, A61P 3/00, A61P 3/04, A61P 3/06, A61P 3/10, A61P 5/00, A61P 5/04, A61P 5/12, A61P 5/46, G01N 33/15, G01N 33/50

(54) **METABOLISM IMPROVING AGENT**

(30) Priority: 25.05.2018 JP 2018100549
(71) Applicant: University of The Ryukyus, Nakagami-gun, Okinawa 903-0213 (JP); Nippon Chemiphar Co., Ltd., Tokyo 101-0032 (JP)
(72) Inventor: MASUZAKI Hiroaki, Nakagami-gun, Okinawa 903-0213 (JP); NAKAMURA Hideki, Misato-shi, Saitama 341-0005 (JP); HIRAI Toshitake, Misato-shi, Saitama 341-0005 (JP); HARA Kaoru, Misato-shi, Saitama 341-0005 (JP); KANDA Takashi, Tokyo 101-0032 (JP); YAMASAKI Satomi, Tokyo 101-0032 (JP); KOBAYASHI Tadashi, Tokyo 101-0032 (JP); NISHIOKA Koichiro, Tokyo 101-0032 (JP)
(74) Representative: Godemeyer Blum Lenze Patentanwälte Partnerschaft mbB - werkpatent
(86) International application number: PCT/JP2019/020565
(87) International publication number: WO 2019/225722

(57) **Abstract**

Provided is a metabolism improving agent that contains, for example, an alkalizing agent such as an acidosis improving agent or a urinary alkalizing agent as an active ingredient, and has actions such as improvement of insulin resistance, improvement of pituitary and adrenal functions, and reduction of visceral fat accumulation.

## Description

### Technical Field

The present invention relates to a metabolism improving agent having actions such as improvement of insulin resistance, improvement of pituitary and adrenal functions, and reduction of visceral fat accumulation.

### Background Art

In recent years, an increase in obesity and visceral fat due to westernization of diet, as well as lifestyle-related diseases (hypertension, hyperlipidemia, diabetes, hyperuricemia, and the like) thought to be accompanied therewith and metabolic syndrome in which they are accumulated have become social problems, and financial burden on medical care has been rapidly increasing.

Although obesity caused by human diet is mainly caused by excessive intake of fat and protein, mechanisms leading to visceral fat accumulation are thought to be different from each other, and in actual obesity pathology, it is imagined that both are complexly intertwined.

For example, when visceral fat is accumulated by excessive intake of fat, it is thought that, acidification of body fluids (acidosis, aciduria) is not observed and weight gain and fat weight increase simultaneously occur, so that fat which has no place to go due to excessive intake of fat undergoes a passive process to be accumulated in the viscera.

On the other hand, it is characterized in that visceral fat accumulation due to excessive protein intake is caused by excessive intake of animal protein as a result of insufficient intake of vegetables and increased intake of animal protein, acidification of body fluids (acidosis, aciduria) is observed, and fat weight increases prior to weight gain. Since glucocorticoids increase at that time, it is thought that they undergo an active process in which a mechanism of a living body that tries to store fat for survival is switched on.

Conventionally, a mechanism of deterioration of obesity and metabolic syndrome by high-fat diet has been studied in many models. However, a dietary model capable of reproducing visceral fat accumulation by a high-protein diet has not been proposed, and research on visceral fat accumulation caused by excessive protein intake has not been advanced. A dietary model (Ann. Nutr. Metab., 50, p.299, 2006) with a high load of casein which is an animal protein has been known, and four types of feeds with different amounts of casein and potassium (13LK: 13% casein + Low (0.3%) K; 13HK: 13% casein + High (2%) K; 26LK: 26% casein + Low (0.3%) K; and 26HK: 26% casein + High (2%) K) have been proposed. However, this model has been proposed for study of acidosis, and has not been used for analysis of the mechanisms leading to visceral fat accumulation, a mechanism for suppressing it, and evaluation of medicines.

### Citation List

### Non Patent Literature

Non Patent Literature 1: Ann. Nutr. Metab., 50, p.299, 2006

### Summary of Invention

### Technical Problem

An object of the present invention is to provide a metabolism improving agent having actions such as improvement of insulin resistance, improvement of pituitary and adrenal functions, reduction of visceral fat accumulation (e.g., suppression of weight increase of mesenteric adipose tissue, suppression of enlargement of mesenteric adipocytes), suppression of acidification of body fluids (e.g., blood, urine), decrease in blood sugar level, decrease in neutral fat (e.g., triglyceride) level in the blood, decrease in uric acid level in the blood and normalization of enhancement of hypothalamic-pituitary-adrenocortical system (HPA axis), and more specifically, to provide a medicine and food having an action for improving metabolic disorder caused by excessive protein intake by mainly eating meat.

Further, another object of the present invention is to provide an experimental model animal capable of reproducing visceral fat accumulation due to high protein load, which can be used for analysis of the mechanisms of increase in visceral fat due to excessive protein intake and deterioration of metabolic syndrome.

### Solution to Problem

In order to solve the above-mentioned problems, the present inventors have found that, among the feeds used in a high casein-loaded rat model (Ann. Nutr. Metab., 50, p. 299, 2006) that is a dietary model of a high protein diet proposed as a model reproducing acidosis pathology, rats fed with feed (13LK) that combined 13% casein and low potassium (0.3%) can be used as a dietary model rat that can reproduce visceral fat accumulation caused by lack of vegetables and excessive animal protein intake by mainly eating meat. In this model rat, mesenteric adipocytes become significantly larger as compared to a normal diet (a vegetable soy protein based diet) by one week of feeding.

Furthermore, the present inventors have conducted intensive studies using the above-mentioned model rats to provide a metabolism improving agent having actions such as improvement of insulin resistance, improvement of pituitary and adrenal functions, and reduction of visceral adipose tissue, and found that an alkalizing agent, preferably citrate, has actions for improving metabolic disorder such as improvement of insulin resistance, improvement of pituitary and adrenal functions, reduction of visceral fat accumulation (e.g., suppression of weight increase of mesenteric adipose tissue, suppression of enlargement of mesenteric adipocytes), suppression of acidification of body fluids (e.g., blood, urine), decrease in blood sugar level, decrease in uric acid level in the blood, decrease in neutral fat (e.g., triglyceride) level in the blood and normalization of enhancement of hypothalamic-pituitary-adrenocortical system (HPA axis), thus is useful as a metabolism improving agent, and that the above metabolism improving agent is effective particularly for improvement of metabolic disorder caused by excessive intake of animal protein by mainly eating meat, for example, improvement of metabolic syndrome, and accomplished the present invention.

That is, the present invention provides a metabolism improving agent containing an alkalizing agent (e.g., an acidosis improving agent, a urinary alkalizing agent) as an active ingredient.

According to a preferred embodiment of the present invention, there are provided the metabolism improving agent in which the alkalizing agent is citrate; the metabolism improving agent containing citrate as an active ingredient; the metabolism improving agent containing a mixture of sodium citrate and potassium citrate as an active ingredient; and the metabolism improving agent containing a mixture of sodium citrate hydrate and potassium citrate as an active ingredient.

According to a further preferred embodiment of the present invention, there are provided the metabolism improving agent in which metabolic improvement is improvement of insulin resistance; the metabolism improving agent in which metabolic improvement is pituitary function improvement (e.g., suppression of increase in blood ACTH); the metabolism improving agent in which metabolic improvement is adrenal function improvement (e.g., suppression of increase in blood and/or urinary glucocorticoid (e.g., cortisol)); the metabolism improving agent in which metabolic improvement is normalization of enhancement of hypothalamic-pituitary-adrenocortical system (HPA axis) (e.g., suppression of secretion of glucocorticoids (e.g., cortisol) from the adrenal cortex; decrease in blood glucocorticoid (e.g., cortisol) level, relative to a ratio of blood glucocorticoid (e.g., cortisol) level to blood ACTH level (blood glucocorticoid (e.g., cortisol) level/blood ACTH level); increase in a ratio of blood ACTH level to blood glucocorticoid (e.g., cortisol) level (blood ACTH level/blood glucocorticoid (e.g., cortisol) level), relative to blood glucocorticoid (e.g., cortisol) level); the metabolism improving agent in which metabolic improvement is reduction of visceral fat accumulation (e.g., suppression of weight increase of mesenteric adipose tissue, suppression of enlargement of mesenteric adipocytes); the metabolism improving agent in which metabolic improvement is suppression of acidification of body fluids (e.g., blood, urine); the metabolism improving agent in which metabolic improvement is decrease in blood sugar level; the metabolism improving agent in which metabolic improvement is decrease in neutral fat (e.g., triglyceride) level in the blood; the metabolism improving agent in which metabolic improvement is decrease in uric acid level in the blood; the metabolism improving agent in which metabolic improvement is improvement of metabolic disorder caused by excessive protein intake; the metabolism improving agent in which metabolic improvement is improvement of metabolic disorder in obesity caused by excessive protein intake; the metabolism improving agent in which metabolic improvement is prevention and/or improvement of metabolic syndrome; the metabolism improving agent in which metabolic improvement is prevention and/or improvement of metabolic acidosis; the metabolism improving agent used for preventing and/or improving obesity by improvement of metabolic disorder in obesity caused by excessive protein intake; the metabolism improving agent which is a medicine; and the metabolism improving agent which is a food.

In another aspect, the present invention provides use of an alkalizing agent, preferably citrate, for production of the metabolism improving agent; and a method for improving metabolism in a mammal including human, including administering an effective amount of citrate to a mammal including human.

From still another viewpoint, the present invention provides a method for preparing a model animal with visceral fat accumulation due to insufficient intake of vegetables and excessive intake of animal protein, including feeding rats a diet combining 13% casein and 0.3% potassium. In a preferred embodiment of the invention, the animal is a rat, and feeding can be continued, preferably for 1 to 4 weeks.

Further, the present invention provides a method for screening an improving agent for metabolic disorder caused by excessive protein intake using the model animal, preferably a model rat; a method for screening a medicine having a preventive and/or therapeutic action on metabolic syndrome on metabolic syndrome using the model animal, preferably a model rat; and a method for determining effectiveness of an improving agent for metabolic disorder caused by excessive protein intake or a medicine having a preventive and/or therapeutic action on metabolic syndrome using the model animal, preferably a model rat.

### Advantageous Effects of Invention

The metabolism improving agent of the present invention has actions for improving metabolic disorder such as improvement of insulin resistance, pituitary function improvement (e.g., suppression of increase or decrease in blood ACTH level), and improvement of adrenal function (e.g., suppression of increase or decrease in blood and/or urinary glucocorticoid (e.g., cortisol) level), normalization of enhancement of hypothalamic-pituitary-adrenocortical system (HPA axis) (e.g., suppression of glucocorticoid (e.g., cortisol) secretion from the adrenal cortex; decrease in blood glucocorticoid (e.g., cortisol) level, relative to a ratio of blood glucocorticoid (e.g., cortisol) level to blood ACTH level (blood glucocorticoid (e.g., cortisol) level/blood ACTH level); increase in a ratio of blood ACTH level to blood glucocorticoid (e.g., cortisol) level (blood ACTH level/blood glucocorticoid (e.g., cortisol) level), relative to blood glucocorticoid (e.g., cortisol) level); reduction of visceral fat accumulation (e.g., suppression of weight increase of mesenteric adipose tissue, suppression of enlargement of mesenteric adipocytes), suppression of acidification of body fluids (e.g., blood, urine), decrease or suppression of increase in blood sugar level, suppression of increase or decrease in blood insulin level, suppression of increase or decrease in uric acid level in the blood, and decrease in neutral fat (e.g., triglyceride) level in the blood, and, for example, is useful as an improving agent for metabolic disorder caused by excessive protein intake, and is particularly effective for improving metabolic disorder caused by insufficient intake of vegetables and excessive intake of animal protein and preventing and/or treating metabolic syndrome. In addition, the metabolism improving agent of the present invention is useful for improving metabolic disorder caused by excessive protein intake, preferably, insufficient intake of vegetables and excessive intake of animal protein and preventing and/or treating metabolic syndrome.

In addition, the model animal provided by the present invention, preferably a model rat, is a model animal capable of reproducing obesity due to metabolic disorder, for example, increase in visceral fat (e.g., increase in weight of mesenteric adipose tissue, increase in size of mesenteric adipose tissue), a model animal capable of inducing insulin resistance, a model animal capable of inducing pituitary dysfunction (e.g., increase in blood ACTH), a model animal capable of inducing abnormality of adrenal function (e.g., increase in blood and/or urinary glucocorticoid (e.g., cortisol)), a model animal capable of inducing enhancement of the hypothalamic-pituitary-adrenocortical system (HPA axis) (e.g., increased secretion of glucocorticoids (e.g., cortisol) from the adrenal cortex; decrease in blood glucocorticoid (e.g., cortisol) level, relative to a ratio of blood ACTH level to blood glucocorticoid (e.g., cortisol) level (blood ACTH level/blood glucocorticoid (e.g., cortisol) level); increase in a ratio of blood glucocorticoid (e.g., cortisol) level to blood ACTH level (blood glucocorticoid (e.g., cortisol) level/blood ACTH level), relative to blood glucocorticoid (e.g., cortisol) level), a model animal capable of inducing acidification of body fluids (e.g., blood, urine), a model animal capable of inducing increase in blood insulin level, a model animal capable of inducing increase in blood leptin level, a model animal capable of inducing increase in blood uric acid level and/or a model animal capable of inducing abnormal blood sugar level.

Preferably, it is a dietary model animal capable of reproducing a state of metabolic disorder caused by insufficient intake of vegetables and excessive intake of animal protein by mainly eating meat, a model animal capable of reproducing increase in visceral fat accompanied by metabolic disorder caused by insufficient intake of vegetables and excessive intake of animal protein (e.g., increase in weight of mesenteric adipose tissue, enlargement of mesenteric adipocytes), a model animal capable of reproducing a state of metabolic syndrome, a model animal that can be used for screening of a medicine having an action for improving metabolic disorder caused by excessive protein intake, particularly, metabolic disorder caused by insufficient intake of vegetables and excessive intake of animal protein and effectiveness confirmation of the medicine, as well as elucidation of mechanism of visceral fat accumulation (e.g., increase in weight of mesenteric adipose tissue, enlargement of mesenteric adipocytes) caused by excessive animal protein intake, elucidation of onset mechanism of metabolic syndrome, and the like.

### Brief Description of Drawings

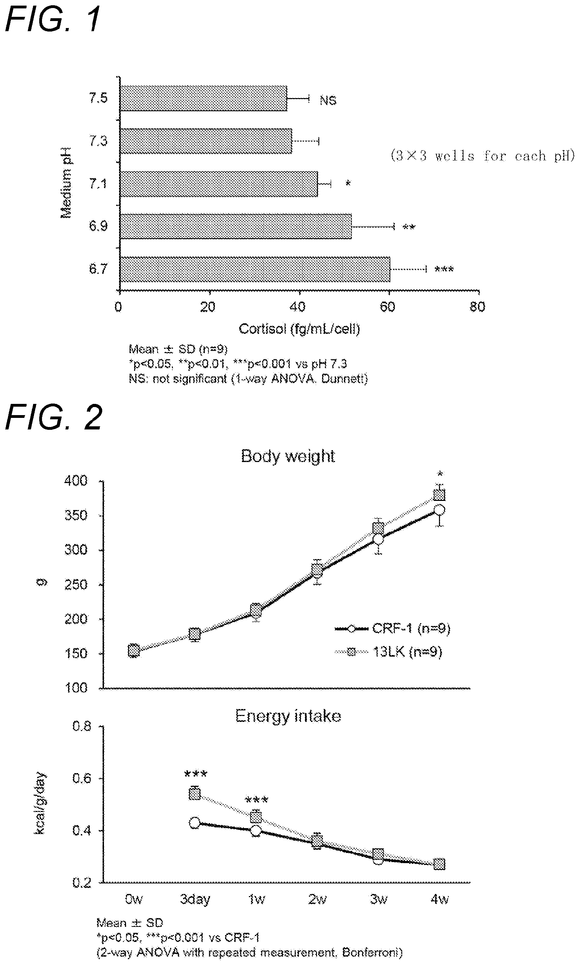

Fig. 1 illustrates a diagram showing results of confirming that release of glucocorticoids (cortisol) was increased by using a human adrenocortical carcinoma cell line and adjusting pH of a culture solution to make environment around the cells acidic. Mean ± SD (n=9), *p < 0.05, **p < 0.01, ***p < 0.001 vs pH 7.3, NS: not significant (1-way ANOVA, Dunnett)
Fig. 2 illustrates diagrams showing changes in body weight and energy intake when rats are freely fed a diet (13LK) combining 13% casein and 0.3% potassium simulating meat diet with lack of vegetables. CRF-1 (standard feed group), Mean ± SD, *p < 0.05, ***p < 0.001 vs CRF-1 (2-way ANOVA with repeated measurement, Bonferroni)

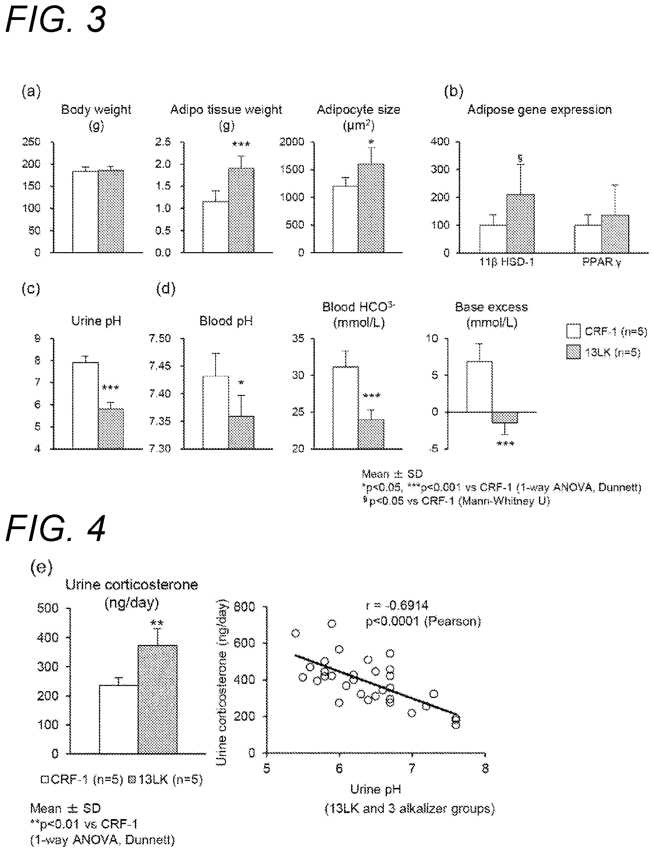

Fig. 3 illustrates diagrams showing changes in (a) body weight, adipose tissue weight, adipocyte size, (b) adipose gene expression, (c) urine pH, (d) blood pH, and (e) blood HCO₃- and Base excess when rats are freely fed a diet (13LK) combining 13% casein and 0.3% potassium simulating meat and vegetable diet. CRF-1 (standard feed group), Mean ± SD, *p < 0.05, ***p < 0.001 vs CRF-1 (1-way ANOVA, Dunnett), ^{§}p < 0.05 vs CRF-1 (Mann-Whitney U) Fig. 4 illustrates diagrams (e) showing a change in daily excretion of urinary glucocorticoids (corticosterone) and a correlation with a change of urine pH when rats are freely fed a diet (13LK) combining 13% casein and 0.3% potassium simulating meat diet with lack of vegetables. CRF-1 (standard feed group). Mean ± SD, **p < 0.01 vs CRF-1 (1-way ANOVA, Dunnett)

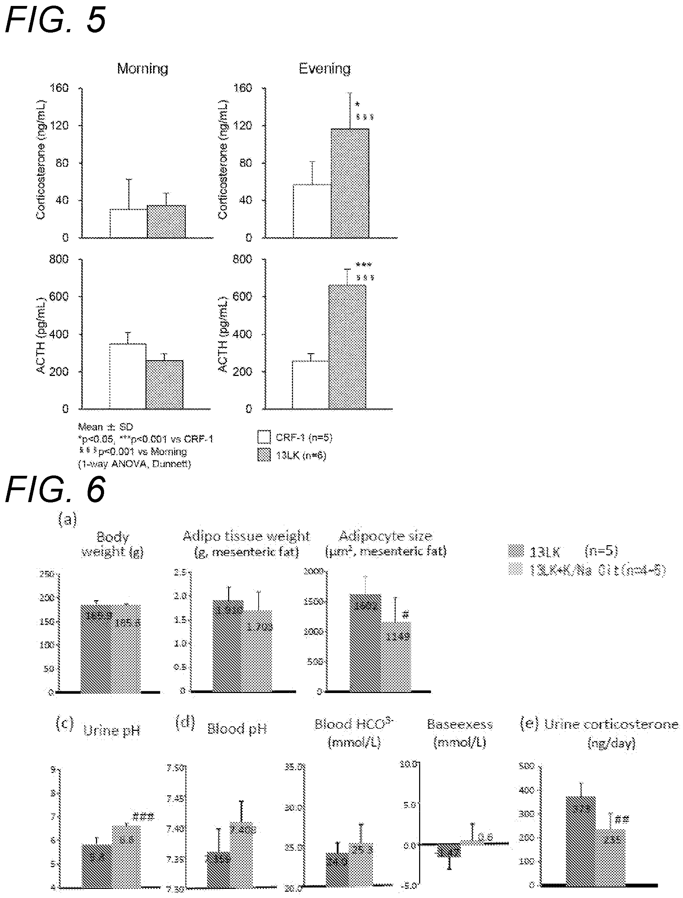

Fig. 5 illustrates diagrams comparing changes in blood glucocorticoid (corticosterone) and blood ACTH between Morning (inactive period) and Evening (active period) in consideration of the daily fluctuation of both parameters when rats are freely fed a diet (13LK) combining 13% casein and 0.3% potassium simulating meat diet with lack of vegetables. CRF-1 (standard feed group), Mean ± SD, *p < 0.05, ***p < 0.001 vs CRF-1, ^{§§§}p < 0.001 vs Morning (1-way ANOVA, Dunnett)
Fig. 6 illustrates diagrams showing results of confirming an action of citrate using a model rat of the present invention (13LK was freely fed as a feed for one week). 13LK (drug non-administration group), 13LK + K/NaCit (citrate administration group), Mean ± SD, ^{#}p < 0.05, ^{##}p < 0.01, ^{###}p < 0.001 vs 13LK (1-way ANOVA, Dunnett)

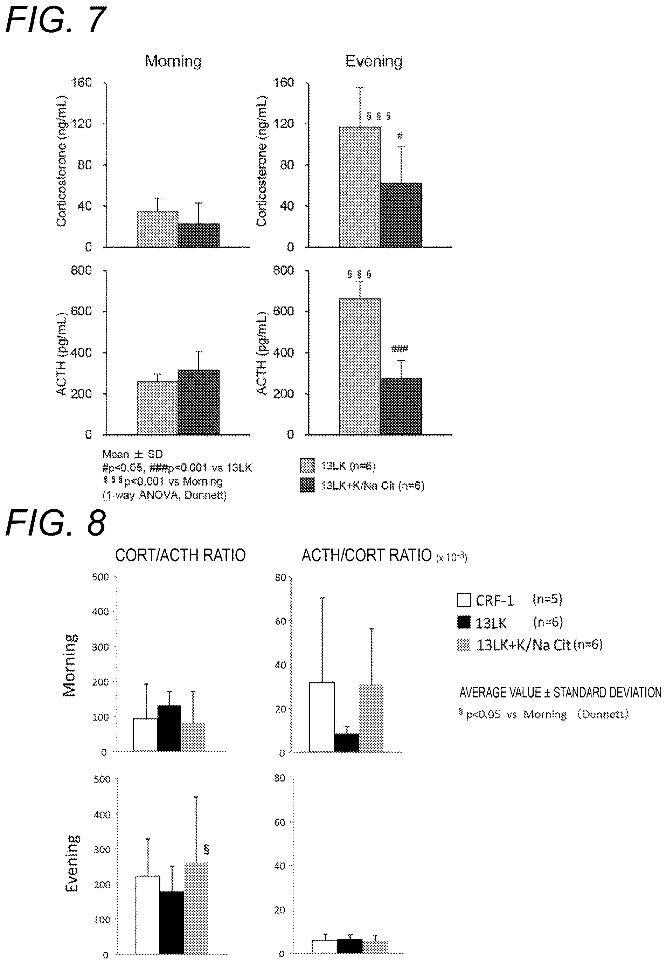

Fig. 7 illustrates diagrams showing results of confirming an action of citrate using a model rat of the present invention (13LK was freely fed as a feed for one week). 13LK (drug non-administration group), 13LK + K/Na Cit (citrate administration group), Mean ± SD, ^{#}p < 0.05, ^{###}p < 0.001 vs 13LK, ^{§§§}p < 0.001 vs Morning (1-way ANOVA, Dunnett)
Fig. 8 illustrates diagrams comparing a ratio of glucocorticoid (corticosterone: CORT) concentration in the blood to ACTH concentration (CORT/ACTH and ACTH/CORT) in the blood between Morning (inactive period) and Evening (active period) in a model rat of the present invention (13LK was freely fed as a feed for one week), a rat administered with citrate to a model rat of the present invention, and a rat freely fed a standard feed as a feed for one week. CRF-1 (standard feed group), 13LK (drug non-administration group), 13LK + K/Na Cit (citrate administration group), Mean ± SD, ^{§}p < 0.05 vs Morning (1-way ANOVA, Dunnett)

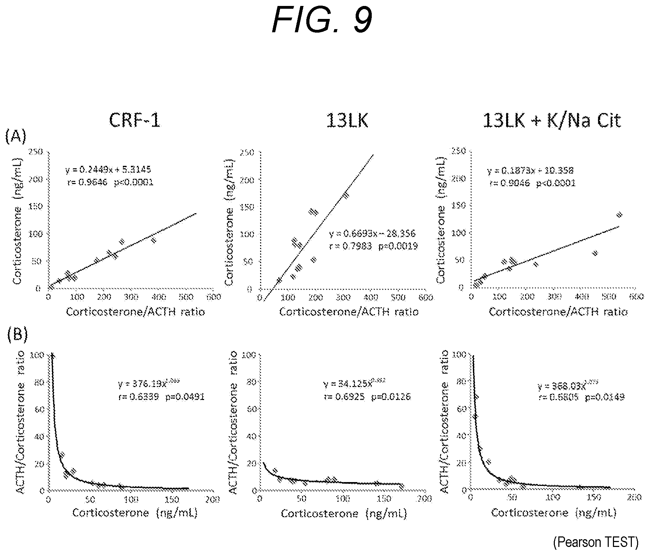

Fig. 9 illustrates diagrams showing a correlation between a ratio of glucocorticoid (corticosterone: CORT) concentration in the blood to ACTH concentration (CORT/ACTH and ACTH/CORT) in the blood and corticosterone in a model rat of the present invention (13LK was freely fed as a feed for one week), a rat administered with citrate to a model rat of the present invention, and a rat freely fed a standard feed as a feed for one week. CRF-1 (standard feed group), 13LK (drug non-administration group), 13LK + K/Na Cit (citrate administration group)

### Description of Embodiments

The metabolism improving agent of the present invention is characterized by containing an alkalizing agent as an active ingredient. As the alkalizing agent, for example, citrate, sodium bicarbonate or the like can be used, but it is not limited thereto. A mixture of two or more alkalizing agents may be used. As the citrate, for example, sodium citrate (e.g., C₆H₅Na₃O₇ · 2H₂O), potassium citrate (e.g., C₆H₅K₃O₇ · H₂O) or the like can be used, but it is not limited thereto. The citrate salt may be any hydrate or solvate.

In addition, any mixture of two or more citrates can be used as an alkalizing agent as an active ingredient of the medicine of the present invention. For example, a mixture of sodium citrate and potassium citrate can be also used. In this case, for example, a combination of sodium citrate hydrate and potassium citrate may be used. As a medicine containing such an active ingredient, a tablet containing 231.5 mg of potassium citrate and 195.0 mg of sodium citrate hydrate in one tablet can be also used as the metabolism improving agent of the present invention.

In one embodiment, the metabolism improving agent provided by the present invention may contain citric acid (e.g., anhydrous citric acid), in addition to citrate (e.g., sodium citrate and/or potassium citrate).

The metabolism improving agent of the present invention has metabolism improving actions such as improvement of insulin resistance, pituitary function improvement, improvement of adrenal functions, normalization of enhancement of hypothalamic-pituitary-adrenocortical system (HPA axis), reduction of visceral fat accumulation, suppression of acidification of body fluids (e.g., blood, urine), decrease in blood sugar level, decrease in uric acid level in the blood, and decrease in neutral fat (e.g., triglyceride) level in the blood. The action of the metabolism improving agent of the present invention can be confirmed, for example, by a method specifically shown in Examples of the present specification. More specifically, by using a model animal of the present invention capable of reproducing a state of increased visceral fat caused by a decrease in vegetable intake and an increase in animal protein intake by mainly eating meat, preferably a model rat, it is possible to confirm improvement of insulin resistance, pituitary function improvement, improvement of adrenal functions, normalization of enhancement of hypothalamic-pituitary-adrenocortical system (HPA axis), reduction of visceral fat accumulation, suppression of acidification of body fluids (e.g., blood, urine), decrease in blood sugar level, decrease in blood insulin level, decrease in blood leptin level, decrease in uric acid level in the blood, decrease in neutral fat (e.g., triglyceride) level in the blood, and the like.

The metabolism improving agent of the present invention can be used, for example, as an improving agent for metabolic disorder caused by excessive protein intake. Preferably, it is effective for improving metabolic disorder caused by insufficient intake of vegetables and excessive intake of animal protein, and for preventing and/or treating metabolic syndrome. Metabolic syndrome is a pathological condition that exhibits two symptoms out of following i) to iii): i) abnormal serum lipid, ii) high blood pressure value, and iii) hyperglycemia, in addition to visceral fat accumulation. In addition, the metabolism improving agent of the present invention can also be used for prevention and/or treatment of obesity caused by excessive protein intake, by improving metabolic disorder caused by excessive protein intake, preferably, insufficient intake of vegetables and excessive intake of animal protein.

A subject to which the metabolism improving agent of the present invention is administered may be a person suffering from metabolic syndrome, a person with accumulation of visceral fat, a person with high visceral fat level, or a person suffering from metabolic acidosis. For example, the subject to which the metabolism improving agent of the present invention is administered may be a person whose urine has been acidified (e.g., a person with a urine pH of 4.0 or more and less than 6.2) or a person with high uric acid level (e.g., a person with a blood uric acid level of 6.0 to 7.0 mg/dL, a person with a blood uric acid level of 7.0 mg/dL or more), a person with high blood pressure (e.g., person with a systolic blood pressure of 130 to 180 mmHg or a diastolic blood pressure of 85 to 109 mmHg), a person with high blood sugar level (e.g., a person with a fasting blood sugar level of 100 mg/dL to 126 mg/dL or 126 mg/dL or more), or a person with high blood neutral fat level (e.g., a person with a blood neutral fat level of 100 mg/dL to 149 mg/dL or 150 mg/dL or more). As a result of administering the metabolism improving agent of the present invention, the metabolism improving agent of the present invention can exhibit metabolism improving actions such as improvement of insulin resistance or suppression of exacerbation of insulin resistance, pituitary function improvement, improvement of adrenal functions, normalization of enhancement of hypothalamic-pituitary-adrenocortical system (HPA axis), reduction of visceral fat accumulation, suppression of acidification of body fluids (e.g., blood, urine), decrease in blood sugar level or suppression of increase in blood sugar level, decrease in uric acid level in the blood or suppression of increase in uric acid level in the blood, and decrease in neutral fat (e.g., triglyceride) level in the blood. The visceral fat level can be appropriately determined by those skilled in the art, for example, using a weight and body composition meter manufactured by OMRON Corporation, according to the criteria of the company.

A method of administering the medicine of the present invention is not particularly limited, but it can be generally administered orally using solid preparations such as tablets and capsules, or solutions, suspensions, syrups, or the like. Parenteral preparations such as intravenous preparations can be also used as necessary. Tablets may contain pharmaceutically acceptable additives (e.g., excipients, disintegrants, binders, lubricants) in addition to the alkalizing agent, and may be produced by a method known in the pharmaceutical field. For example, the tablets may be produced by a method including mixing an alkalizing agent with an excipient (e.g., lactose, D-mannitol, crystalline cellulose, glucose), a disintegrant (e.g., starch, calcium carboxymethylcellulose (CMC-Ca)), a binder (e.g., hydroxypropylcellulose (HPC), gelatin, polyvinylpyrrolidone (PVP)), a lubricant (e.g., magnesium stearate, talc), and/or a stabilizer (citric anhydride), and tableting the mixture. The tablets may be uncoated tablets or film-coated tablets.

A dose of the medicine of the present invention is not also particularly limited, but it is known that urinary acidification progresses due to excessive protein intake, and urinary acidification may be an indicator of metabolic disorder due to excessive protein intake. Therefore, it is generally preferred to select a dose required to improve urinary acidification, preferably urinary alkalinization (e.g., urine pH that falls in the range of pH 6.5 or more in urine tests). For example, when citrate is used as a urinary alkalizing agent, the dose can be set to 1 to 10 g (e.g., 1 to 6 g) per day and preferably about 3 to 6 g per day as a potassium citrate/sodium citrate hydrate compounding agent, but is not limited to this dose, and can be appropriately increased or decreased according to the type of metabolic disorder to be improved, weight and age of a patient, and the like.

The active ingredient of the metabolism improving agent of the present invention may be used as a food. The food provided by the present invention is useful, for example, as a food having an improving effect on metabolic disorder caused by excessive protein intake, or as a food having an improving effect on metabolic disorder caused by acidification of body fluids.

The food can be taken by humans or other mammals (e.g., healthy humans, healthy mammals) to an extent that it is not intended to treat or prevent a disease. Examples of subjects who take the food provided by the present invention include healthy persons who tend to be deficient in vegetable intake, healthy persons who tend to eat a diet high in protein, healthy persons who tend to take in a large amount of protein, healthy persons who tend to be deficient in vegetable intake and tend to eat a diet high in protein, healthy persons who tend to be deficient in vegetable intake and tend to take in a large amount of protein, healthy persons who are deficient in vegetable intake, healthy persons who take excessive animal protein, healthy persons who are deficient in vegetable intake and take excessive animal protein, healthy persons with high visceral fat level, healthy persons with accumulation of visceral fat, somewhat obese persons (e.g., persons with a BMI of 23 to 30, or persons with a BMI of 25 to 30), healthy persons with visceral fat obesity, healthy persons whose body fluids are acidified (e.g., healthy persons whose urine is acidified (e.g., healthy persons with a urine pH of 4.5 to 6.0, 5.0 to 6.0 or 5.5 to 6.0)), healthy persons with relatively high blood pressure (e.g., healthy persons with a systolic blood pressure of 130 mmHg to 139 mmHg or a diastolic blood pressure of 85 mmHg to 89 mmHg), healthy persons with relatively high blood sugar levels (e.g., healthy persons with normal-high fasting blood sugar levels (e.g., 100 mg/dL to 110 mg/dL), healthy persons with relatively high blood neutral fat levels (e.g., healthy persons with blood neutral fat levels of 100 mg/dL to 149 mg/dL), and healthy persons with relatively high uric acid levels (e.g., healthy persons with blood uric acid levels of 6.0 to 7.0 mg/dL). Further examples of the subjects who take the food provided by the present invention include persons (e.g., healthy persons) having at least one of characteristics consisting of following i) to xi): i) excessive protein (e.g., animal protein) intake, ii) high visceral fat level, iii) accumulation of visceral fat, iv) somewhat obese (e.g., BMI of 23 to 30 or BMI of 25 to 30), v) visceral fat obesity, vi) body fluids are acidified (e.g., urine pH of 4.5 to 6.0, 5.0 to 6.0 or 5.5 to 6.0), vii) relatively high blood pressure (e.g., a systolic blood pressure of 130 mmHg to 139 mmHg or a diastolic blood pressure of 85 mmHg to 89 mmHg), viii) relatively high blood sugar levels (e.g., normal-high fasting blood sugar levels (e.g., 100 mg/dL to 110 mg/dL)), ix) relatively high blood neutral fat levels (e.g., blood neutral fat levels of 100 mg/dL to 149 mg/dL), x) relatively high uric acid levels (e.g., blood uric acid levels of 6.0 to 7.0 mg/dL), and xi) insufficient intake of vegetables. Whether or not the protein intake is large may be referred to, for example, Dietary Reference Intakes for Japanese (2015 version) (Ministry of Health, Labor and Welfare).

By taking the food provided by the present invention, the same effects as those of the metabolism improving agent of the present invention can be obtained. Examples of effects obtained by taking the food provided by the present invention include improvement of insulin resistance or suppression of exacerbation of insulin resistance, pituitary function improvement (e.g., suppression of increase or decrease in blood ACTH level), and improvement of adrenal function (e.g., suppression of increase or decrease in blood and/or urinary glucocorticoid (e.g., cortisol) level), normalization of enhancement of hypothalamic-pituitary-adrenocortical system (HPA axis) (e.g., suppression of glucocorticoid (e.g., cortisol) secretion from the adrenal cortex; decrease in blood glucocorticoid (e.g., cortisol) level, relative to a ratio of blood glucocorticoid (e.g., cortisol) level to blood ACTH level (blood glucocorticoid (e.g., cortisol) level/blood ACTH level); increase in a ratio of blood ACTH level to blood glucocorticoid (e.g., cortisol) level (blood ACTH level/blood glucocorticoid (e.g., cortisol) level), relative to blood glucocorticoid (e.g., cortisol) level); reduction of visceral fat accumulation (e.g., suppression of weight increase of mesenteric adipose tissue, suppression of enlargement of mesenteric adipocytes), suppression of acidification of body fluids (e.g., blood, urine), decrease in blood sugar level or suppression of increase in blood sugar level, decrease in uric acid level in the blood or suppression of increase in uric acid level in the blood, decrease in insulin level in the blood or suppression of increase in insulin level in the blood, decrease in leptin level in the blood or suppression of increase in leptin level in the blood, decrease in neutral fat (e.g., triglyceride) level in the blood, and combinations thereof.

The form of the food is not particularly limited as long as it can be taken orally, and may be the form of a supplement or a general food. Examples of general food include drinks (e.g., drinks containing fruit juice such as juice or vegetable extract, tea drinks, sports drinks, near water, diet drinks), candy, jelly, gummy, and gum. The food provided by the present invention can be appropriately produced by those skilled in the art depending on the type of the food, and for example, may be produced by adding an alkalizing agent to a food material. The content of the alkalizing agent contained in the food can also be appropriately set by those skilled in the art. For example, when using a mixture of potassium citrate and sodium citrate hydrate as the alkalizing agent, potassium citrate and sodium citrate hydrate may be contained in the food to take potassium citrate and sodium citrate hydrate in a total of 1 to 10 g (e.g., 1 to 6 g), and preferably 1 to 3 g per day. When the food provided by the present invention is a tablet supplement, for example, 300 mg to 600 mg tablets may be produced to contain 70 to 80% by weight of an alkalizing agent (e.g., potassium citrate and sodium citrate hydrate) per tablet, according to the tablet production method for the above medicine. When the alkalizing agent is potassium citrate and/or sodium citrate hydrate, the food provided by the present invention may further contain citric acid (e.g., citric anhydride).

Examples of embodiments provided by the present invention include:
(1) A metabolism improving agent containing an alkalizing agent as an active ingredient.
(2) The metabolism improving agent according to (1), wherein the alkalizing agent is citrate or sodium bicarbonate.
(3) The metabolism improving agent according to (1), wherein the alkalizing agent is citrate or a mixture of two or more citrates.
(4) The metabolism improving agent according to any one of (1) to (3), wherein metabolic improvement is improvement of metabolic disorder caused by excessive protein intake.
(5) The metabolism improving agent according to any of (1) to (3), wherein metabolic improvement is improvement of metabolic disorder in obesity caused by excessive protein intake.
(6) The metabolism improving agent according to any one of (1) to (3), wherein metabolic improvement is improvement of metabolic disorder in metabolic syndrome.
(7) The metabolism improving agent according to any one of (1) to (3), wherein metabolic improvement is improvement of metabolic disorder in metabolic acidosis.
(8) The metabolism improving agent according to any one of (1) to (7), which is used for preventing and/or treating obesity by improving metabolic disorder in obesity caused by excessive protein intake.
(9) The metabolism improving agent according to any one of (1) to (8), wherein the metabolism improving agent is an insulin resistance improving agent, a body fluid acidification inhibitor, a hypoglycemic agent, a visceral fat accumulation reducing agent, and/or a blood neutral fat (e.g., triglyceride) level-lowering agent.
(10) The metabolism improving agent according to any one of (1) to (9), wherein the metabolism improving agent is a blood ACTH increase inhibitor or a blood and/or urinary glucocorticoid increase inhibitor.
(11) The metabolism improving agent according to any one of (1) to (9), wherein the metabolism improving agent is a blood ACTH lowering agent or a blood and/or urinary glucocorticoid lowering agent.
(12) The metabolism improving agent according to any one of (1) to (11), wherein the metabolism improving agent is a normalizing agent for enhancement of hypothalamic-pituitary-adrenocortical system (HPA axis).
(13) The metabolism improving agent according to (12), wherein normalization of enhancement of the hypothalamic-pituitary-adrenocortical system (HPA axis) is suppression of glucocorticoid secretion from the adrenal cortex; increase in a ratio of blood ACTH level to blood glucocorticoid level (blood ACTH level/blood glucocorticoid level), relative to blood glucocorticoid level; or decrease in blood glucocorticoid level, relative to a ratio of blood glucocorticoid level to blood ACTH level (blood glucocorticoid level/blood ACTH level).
(14) The metabolism improving agent according to any one of (1) to (13), wherein, as a result of administration or ingestion of a metabolism improving agent to a mammal (e.g., a human), the insulin level in the blood decreases, the uric acid level in the blood decreases, and/or the leptin level in the blood decreases.
(15) The metabolism improving agent according to any one of (1) to (14), which is a food.
(16) The metabolism improving agent according to any one of (1) to (15), which is administered or ingested to a person with high visceral fat level.
(17) The metabolism improving agent according to any one of (1) to (16), which is administered or ingested to a person having a characteristic of following i) to vi): i) BMI of 23 or more and 30 or less, ii) relatively high blood sugar level, iii) relatively high blood pressure, iv) relatively high blood neutral fat level, v) relatively high blood uric acid level, or vi) a combination of i) to v).
(18) The metabolism improving agent according to any one of (1) to (17), which is administered or ingested to a person whose body fluids are acidified (e.g., a person whose body fluids (e.g., urine) are acidified due to excessive intake of animal protein).
(19) A method for preparing a model animal with visceral fat accumulation due to excessive intake of animal protein and insufficient intake of vegetables (low potassium load), wherein rats are fed a diet combining 13% casein and 0.3% potassium.
(20) A method for screening an improving agent for metabolic disorder caused by excessive protein intake using the model animal as defined in (19).
(21) A method for determining an efficacy of an improving agent for metabolic disorder caused by excessive protein intake using the model animal as defined in (19).

The model animal provided by the present invention can be used for, as a model animal that reproduces accumulation of visceral fat accompanied by metabolic disorder caused by insufficient intake of vegetables and excessive intake of animal protein, for example, metabolic disorder caused by excessive protein intake, particularly, a medicine having an improving effect on metabolic disorder caused by insufficient intake of vegetables and excessive intake of animal protein, a screening of a medicine having a preventive and/or therapeutic action on metabolic syndrome, and effectiveness confirmation of the medicine. However, the use of the model animal of the present invention is not limited to the specific uses described above. As the model animal of the present invention, a model rat can be preferably used. When a rat is used, the model rat of the present invention can be prepared by freely feeding a diet combining 13% casein and 0.3% potassium usually for 1 to 4 weeks. The period can be appropriately changed depending on factors such as degrees of target visceral adipose tissue amount, acidity of body fluids (e.g., blood, urine), and blood sugar level and neutral fat (e.g., triglyceride) in the blood.

The model animal provided by the present invention is characterized in that a change occurs in the visceral adipose tissue in a short period of time, as compared to high fat diet-loaded model animals and transgenic model animals that are conventional model animals for obesity research, which require one to several months to develop obesity. In the model animal of the present invention, after feeding for about one week, 11βH3B-1 mRNA expression is increased in the visceral adipose tissue even if there is no difference in body weight from the control group, and an increase in weight and an increase in cell size of the visceral adipose tissue are observed, further, various symptoms of metabolic syndrome such as clear insulin resistance are observed in about 2 weeks. Without being bound by any particular theory, it is thought that a pathogenesis mechanism of the model animal of the present invention is glucocorticoid hypersecretion from the adrenal cortex due to a bias toward the acidic side of the biological environment, and it is thought that, in the early stage of obesity formation, glucocorticoid hypersecretion from the adrenal cortex triggers accumulation of visceral fat and leads to the onset of obesity. It is also understood that administration of an alkalizing agent such as citrate suppresses progression of metabolic syndrome in the model animal of the present invention, which also supports the above obesity onset mechanism.

### Examples

Hereinafter, the present invention will be described more specifically with reference to Examples, but the scope of the present invention is not limited by the following Examples.

### Example 1

Whether or not obesity models can be produced by improving a diet of a high casein-loaded rat model (Ann. Nutr. Metab., 50, p.299, 2006), which is a dietary model of a high protein diet capable of reproducing acidosis pathology was examined by the following method.

Prior to that, since McCarty (McCarty MF. Acid-base balance may influence risk for insulin resistance syndrome by modulating cortisol output. Med. Hypotheses 64: 380-384, 2005) has reported that acidosis promotes production of glucocorticoids, visceral obesity is observed in Gushing's syndrome, and 11β-HSD1, conversion enzyme into glucocorticoid activator, is involved in visceral fat accumulation, in order to clarify the point of action, using a human adrenocortical carcinoma cell line, which is a target cell for glucocorticoid (cortisol) release in vitro, it was confirmed that the release of corticoids was increased by adjusting pH of a culture solution to make environment around the cells acidic (Fig. 1).

Rats (Wistar, male, n = 9, 5 weeks old, Charles River Laboratories Japan, Inc.) were freely fed a diet (13LK) combining 13% casein and 0.3% potassium for 4 weeks. As a control group, a vegetable protein based standard feed (CFR-1, manufactured by Oriental Yeast Co., Ltd.) was similarly fed. No change was observed in body weight after 1 week (Fig. 2), but decrease in urine pH, blood pH, HCO₃-, and Base excess (BE) after 1 week was observed (Figs. 3(c) and (d)). On the other hand, urinary glucocorticoid (corticosterone) increased, and a negative correlation was seen with urine pH (Fig. 4(e)). This result indicates that the change in pH in vivo affects glucocorticoid dynamics in vivo.

In addition, an increase in blood corticosterone was also seen, and an increase in blood ACTH was also observed (active period, Fig. 5). Visceral fat (mesenteric fat) increased in weight and cell size, as compared to the control group (Fig. 3(a)). 11β-HSD1 mRNA expression in the mesenteric fat also increased (Fig. 3(b)). In addition, blood leptin significantly increased such that CRF-1 group (n = 10): 0.38 ± 0.19 ng/mL, 13LK group (n = 10): 2.13 ± 0.31 ng/mL, and increase in blood insulin, blood sugar level and HOMA-R value was also seen, and symptoms of metabolic syndrome were observed (Table 1). An increase in blood uric acid level was also observed.

**[Table 1]**

| Metabolic parameter | CRF-1 (n=10) | 13LK (n=10) |
|---|---|---|
| Serum glucose (mg/dL) | 85 ± 11 | 106 ± 21 * |
| Serum insulin (µU/mL) | 1.6 ± 0.8 | 8.0 ± 6.2 ** |
| HOMA-IR | 0.3 ± 0.2 | 2.2 ± 1.8 ** |
| Serum triglyceride (mg/dL) | 38 ± 34 | 36 ± 36 |
| Serum total cholesterol (mg/dL) | 82 ± 11 | 70 ± 13 |
| Serum uric acid (mg/dL) | 1.5 ± 0.2 | 1.9 ± 0.4 ** |

| | | |
|---|---|---|
| Mean ± SD *p<0.05, **p<0.01 vs CRF-1 (1-way ANOVA, Dunnett) | | |

This result is also consistent with the assumption that glucocorticoids are called survival hormones and are involved in fat accumulation (= avoidance of starvation) given to survive an ice age (starvation state). As described above, the model rat of the present invention prepared using 13LK as a feed was proved to be useful as a new metabolic syndrome model animal, particularly as a model animal for visceral fat accumulation caused by lack of vegetables and excessive intake of animal protein. In addition, a series of processes and pathological mechanisms progressing to visceral obesity and metabolic syndrome starting from bioacidification and the accompanying excessive secretion of glucocorticoids from the adrenal cortex were elucidated.

### Example 2

Using the model rat of the present invention prepared in Example 1 (13LK was freely fed as a feed for one week), metabolic improving actions of citrate were confirmed by the following method. An aqueous solution containing citrate (an aqueous solution containing 370 mg of potassium citrate and 312 mg of sodium citrate hydrate in 100 mL) was prepared and administered to the model rat by drinking water (drug administration group: 13LK + K/Na Cit) for one week, and the result was compared to a drug non-administration group (13LK). No change in body weight was observed between the administration group and the non-administration group in one week (Fig. 6(a)).

When changes in each biochemical parameter were confirmed in the same manner as in Example 1, increase in blood HCO₃⁻ and BE was observed with increase in urine pH and blood pH in the drug administration group (Figs. 6(c) and (d)). Furthermore, in the drug administration group, not only the urinary glucocorticoid (corticosterone) decreased, but also the blood glucocorticoid (corticosterone) decreased, and the blood ACTH also decreased (Evening: active period, Fig. 6(e) and Fig. 7). While mesenteric fat did not change in weight as compared to that in the drug non-administration group, suppression of increase in cell size was observed (Fig. 6 (a)). It did not affect 11β-HSD1 mRNA expression in the mesenteric fat. In addition, blood insulin and HOMA-R value decreased, and blood leptin was such that 13LK group (n = 10): 2.13 ± 0.31 ng/mL, K/Na Cit group (n = 9): 1.20 ± 0.77 ng/mL, in which the drug administration group was significantly decreased as compared to the drug non-administration group. As compared to the non-drug-administration group, the drug-administration group showed decrease in blood triglyceride, decrease in blood sugar and decrease in uric acid level. From the above results, citrate showed metabolic improving actions in various symptoms of metabolic syndrome.

In addition, corticosterone (CORT) secretion ability (CORT/ACTH ratio, CORT concentration required for constant ACTH secretion) and ACTH secretion ability (ACTH/CORT ratio, ACTH concentration required for constant CORT secretion) were compared in three groups (Fig. 8). First, the CORT/ACTH ratio shows a considerably high value in the evening (16:30 to 17:30) as compared to the morning (9:30 to 10:30), and the ACTH/CORT ratio showed a stable low value in the evening as compared to the morning (Fig. 8). Here, for example, when the citrate administration group (13LK + K/Na Cit group) suppresses ACTH secretion from the pituitary, the CORT concentration for secreting the same amount of ACTH needs to be increased, thus the CORT/ACTH ratio would increase. On the other hand, when the citrate administration group (13LK + K/Na Cit group) suppresses CORT secretion from the adrenal cortex, the ACTH concentration for secreting the same amount of CORT needs to be increased, thus the ACTH/CORT ratio would increase. As a matter of fact, the CORT/ACTH ratio does not differ between the three groups in the morning and evening, and the ACTH/CORT ratio does not differ between the three groups in the evening, but in the morning, the 13LK + K/Na Cit group and the CRF-1 group clearly showed high values (many individuals showing high values were seen) as compared to the 13LK group (Fig. 8). Thus, it is thought that a possibility that citrate administration suppresses the ACTH secretion from the pituitary is low, and it is a result of suppressing the CORT secretion from the adrenal cortex.

In fact, among the three groups, when analyzing "actual CORT secretion amount and CORT secretion ability" and "actual CORT secretion amount and ACTH secretion ability", in the upper part (A) of Fig. 9, the inclination of the straight line differs, and it can be understood that "in the 13LK group, even though the CORT secretion ability is low, the actual CORT secretion amount is high, and it returns to the normal group level due to decrease in CORT secretion by citrate administration." Further, in the lower part (B) of Fig. 9, the plot distribution differs depending on the group, and it can be understood that "in 13LK, there is no individual showing a low value of the actual CORT secretion amount, but due to decrease in CORT secretion by citrate administration, an individual with increased ACTH secretion ability appeared, and returned to the normal group level" (Fig. 9). It can be understood that the administration of citrate normalized enhancement of the hypothalamic-pituitary-adrenocortical system (HPA axis) seen in the 13LK group.

**[Table 2]**

| Metabolic parameter | 13LK (n=10) | 13LK +0.8% K/Na Cit (n=9) |
|---|---|---|
| Serum glucose (mg/dL) | 106 ± 21 | 91 ± 20 |
| Serum insulin (µU/mL) | 8.0 ± 6.2 | 3,1 ± 1.5^{#} |
| HOMA-IR | 2.2 ± 1.8 | 0.8 ± 0.5^{#} |
| Serum triglyceride (mg/dL) | 36 ± 36 | 18 ± 17 |
| Serum total cholesterol (mg/dL) | 70 ± 13 | 67 ± 12 |
| Serum uric acid (mg/dL) | 1,9 ± 0.4 | 1.6 ± 0.2 |

| | | |
|---|---|---|
| Mean ± SD #p<0.05 vs 13LK (1·way ANOVA, Dunnett) | | |

### Example 3

Citrate or sodium bicarbonate was administered as an alkalizing agent using the model rat of the present invention prepared in Example 1 (13LK was freely fed as a feed for one week), and the results of confirming urine pH and urinary glucocorticoid (corticosterone) excretion are shown in Table 3. Urine pH increased and corticosterone excretion decreased in both the citrate administration group and the sodium bicarbonate administration group as compared to the control group.

**[Table 3]**

| Test point | Parameter | 13LK (n=8) | 13LK + 0.4% NaHCO₃ (n=8) | 13LK + 0.8% K/Na Cit (n=8) | P value (*1·way ANOVA)* |
|---|---|---|---|---|---|
| 1 week | Urine pH | 5.7 ± 0.2 | 6.8 ± 0.6*** | 6.8 ± 0.6*** | 0.0002 |
| | Urine corticosterone (ng/day) | ± 486 ± 115 | 309 ± 116** | 233 ± 93 | 0.0002 |

| | | | | | |
|---|---|---|---|---|---|
| *p<0.05, **p<0.01, ***p<0.0001 vs 13LK (Dunnett) | | | | | |

## Claims

1. A metabolism improving agent comprising an alkalizing agent as an active ingredient, which is a tablet.

2. The metabolism improving agent according to claim 1, wherein the alkalizing agent is citrate or sodium bicarbonate.

3. The metabolism improving agent according to claim 1, wherein the alkalizing agent is citrate or a mixture of two or more citrates.

4. The metabolism improving agent according to any one of claims 1 to 3, wherein metabolic improvement is improvement of metabolic disorder caused by excessive protein intake.

5. The metabolism improving agent according to any one of claims 1 to 3, wherein metabolic improvement is improvement of metabolic disorder in obesity caused by excessive protein intake.

6. The metabolism improving agent according to any one of claims 1 to 3, wherein metabolic improvement is improvement of metabolic disorder in metabolic syndrome.

7. The metabolism improving agent according to any one of claims 1 to 3, wherein metabolic improvement is improvement of metabolic disorder in metabolic acidosis.

8. The metabolism improving agent according to any one of claims 1 to 7, which is used for preventing and/or treating obesity by improving metabolic disorder in obesity caused by excessive protein intake.

9. The metabolism improving agent according to any one of claims 1 to 8, wherein the metabolism improving agent is an insulin resistance improving agent, a body fluid acidification inhibitor, a hypoglycemic agent, a visceral fat accumulation reducing agent, or a blood neutral fat level-lowering agent.

10. The metabolism improving agent according to any one of claims 1 to 8, wherein the metabolism improving agent is a blood ACTH increase inhibitor or a blood and/or urine glucocorticoid increase inhibitor.

11. The metabolism improving agent according to any one of claims 1 to 8, wherein the metabolism improving agent is a normalizing agent for enhancement of hypothalamic-pituitary-adrenocortical system (HPA axis).

12. The metabolism improving agent according to claim 11, wherein normalization of enhancement of the hypothalamic-pituitary-adrenocortical system (HPA axis) is suppression of glucocorticoid secretion from the adrenal cortex; increase in blood ACTH level/blood glucocorticoid level (ratio of blood ACTH level to blood glucocorticoid level), relative to blood glucocorticoid level; or decrease in blood glucocorticoid level, relative to blood glucocorticoid level/blood ACTH level (ratio of blood glucocorticoid level to blood ACTH level).

13. The metabolism improving agent according to any one of 1 to 12, which is a food.

14. The metabolism improving agent according to any one of 1 to 13, which is administered or ingested to a person with high visceral fat level, a person with aciduria, a person with high blood pressure, a person with high blood sugar level, a person with high blood uric acid level, or a person with high blood neutral fat value.

15. The metabolism improving agent according to any one of claims 1 to 14, further comprising citric anhydride.

16. A method for preparing a model animal with visceral fat accumulation due to excessive intake of animal protein and insufficient intake of vegetables (low potassium load), wherein rats are fed a diet combining 13% casein and 0.3% potassium.

17. A method for screening an improving agent for metabolic disorder caused by excessive protein intake using the model animal as defined in claim 16.

18. A method for determining an efficacy of an improving agent for metabolic disorder caused by excessive protein intake using the model animal as defined in claim 16.
